# EUROPEAN PATENT APPLICATION

(11) **EP 4 799 646 A1**
(43) Date of publication of application: **02.09.2026**
(21) Application number: 24875740.3
(22) Date of filing: 05.09.2024
(51) Int. Cl.: A61K 47/44, A61K 9/48, A61K 31/365

(54) **COMPOSITION AND METHOD FOR INCREASING BIOAVAILABILITY OF ACTIVE INGREDIENT**

(30) Priority: 27.10.2023 US 202363593645 P
(71) Applicant: Everfront Biotech Inc., New Taipei City (TW)
(72) Inventor: LI, Yuan-sheng, New Taipei City Taiwan (CN); TAI, Chia Liang, New Taipei City Taiwan (CN); HARN, Horng-jyh, New Taipei City Taiwan (CN); CHIOU, Tzyy-wen, New Taipei City Taiwan (CN); CHOU, Pei-wen, New Taipei City Taiwan (CN); HU, Cheng-hung, New Taipei City Taiwan (CN)
(74) Representative: Herrero & Asociados, S.L.
(86) International application number: PCT/CN2024/117182
(87) International publication number: WO 2025/086915

(57) **Abstract**

Disclosed are a composition comprising an oily medium system and an active ingredient, and a method for increasing bioavailability of an active ingredient. The method comprises incorporating the active ingredient into an oily medium system, wherein the oily medium system comprises a vegetable oil and/or a component of a vegetable oil, but is free of the following substances: phosphate buffered saline(PBS), polyethylene glycols (PEGs), dimethyl sulfoxide (DMSO), ethanol, polypropylene glycols, polysorbates, ethyl acetate, hydroxyethyl 12-hydroxystearate, and tocopheryl polyethylene glycol succinate (TPGS). The active ingredient is selected form the group consisting of: a compound of formula (I), a pharmaceutically acceptable salt of the compound of formula (I), and combinations thereof: 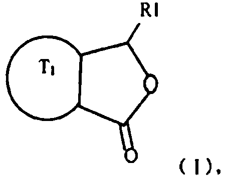 wherein, T₁ is C6 cycloalkane; and R₁ is C1-C8 aliphatic hydrocarbon group.

## Description

### FIELD OF THE INVENTION

The present invention relates to a method for increasing the bioavailability (BA) of an active ingredient, comprising incorporating the active ingredient into an oily medium system. The present invention also relates to a composition comprising the oily medium system and the active ingredient. In the aforementioned method and composition, the oily medium system contains a vegetable oil and/or a component of a vegetable oil, but is free of the following substances: phosphate buffered saline (PBS), polyethylene glycols (PEGs), dimethyl sulfoxide (DMSO), ethanol, polypropylene glycols, polysorbates, ethyl acetate, hydroxyethyl 12-hydroxystearate, and tocopheryl polyethylene glycol succinate (TPGS) Furthermore, the active ingredient is selected from the group consisting of a compound of formula (I), a pharmaceutically acceptable salt of the compound of formula (I), and combinations thereof, 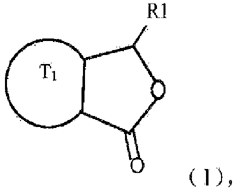 wherein, T₁ is a C6 cycloalkane, and R₁ is a C1-C8 aliphatic hydrocarbon group.

### BACKGROUND OF THE INVENTION

Both brain cancer and pancreatic cancer are highly invasive cancers with high recurrence rates, high mortality rates, and poor prognosis. Brain cancer can occur across all age groups. For common malignant brain tumors such as anaplastic astrocytoma and glioblastoma multiforme (GBM), although traditional surgical treatment can remove most of the tumor masses, it cannot effectively eradicate all cancer cells and must be supplemented by other treatment modalities (such as chemotherapy and radiotherapy). As for pancreatic cancer, due to the frequent occurrence of migration, invasion, and metastasis of pancreatic cancer cells to surrounding nerve or vascular tissues, most patients with pancreatic cancer are already ineligible for surgical treatment at the time of discovery, and therefore, chemotherapy must be used as the prioritized treatment method.

However, chemical drugs currently used clinically for brain cancer or pancreatic cancer remain quite limited, and their therapeutic effects are mostly suboptimal. Therefore, there is still a need for the continuous development of drugs or methods that can effectively treat brain cancer or pancreatic cancer.

Research shows that, in addition to being used to delay or treat neurological diseases such as amyotrophic lateral sclerosis (ALS), spinocerebellar ataxia, and Alzheimer's disease. *n-*butylidenephthalide (BP) can be used to treat cerebrovascular diseases like stroke, to improve diabetes, and to provide efficacy against liver and lung fibrosis. Furthermore, BP possesses excellent cytotoxic effects against brain and pancreatic cancer cells, making it applicable in the field of cancer therapy.

Although *n*-butylidenephthalide possesses the aforementioned medicinal effects, there are still limitations in its application. The primary reason is that hepatic metabolism and excretion would lead to a decrease in the bioavailability of *n*-butylidenephthalide. Consequently, higher doses are often required to achieve the desired therapeutic effect, which increases the risk of systemic side effects and exacerbates the burden on patients. The aforementioned issues arising from the necessity of increasing dosage due to low bioavailability are particularly severe in the case of oral administration. Therefore, effectively improving the bioavailability of the active ingredient would avoid unnecessary dose escalation, resolve application limitations, and provide patients with more effective treatment options.

### SUMMARY OF THE INVENTION

The inventors have discovered that the active ingredient of the present invention (i.e., a compound of formula (I) and/or a pharmaceutically acceptable salt of the compound of formula (I)) has good solubility in the oily medium system of the present invention and can exist stably in said oily medium system for a long period of time (e.g., up to two years when stored at room temperature). The inventors have found from further research that when the active ingredient of the present invention is administered in the form of being incorporated into an oily medium system, the bioavailability of said active ingredient can be increased. This avoids the problem of requiring increased dosage to provide the desired therapeutic benefit, thereby alleviating or eliminating unnecessary side effects.

Therefore, an objective of the present invention is to provide a composition comprising:
(1) an oily medium system, which contains a vegetable oil and/or a component of a vegetable oil, but is free of the following substances: phosphate buffered saline (PBS), polyethylene glycols (PEGs), dimethyl sulfoxide (DMSO), ethanol, polypropylene glycols, polysorbates, ethyl acetate, hydroxyethyl 12-hydroxystearate, and tocopheryl polyethylene glycol succinate (TPGS); and
(2) an active ingredient, which is selected from the group consisting of: a compound of formula (I), a pharmaceutically acceptable salt of the compound of formula (I), and combinations thereof, 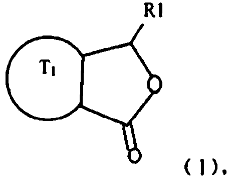 wherein, T₁ is a C6 cycloalkane, and R₁ is a C1-C8 aliphatic hydrocarbon group.

Preferably, the composition is used to increase the bioavailability of the active ingredient. It is preferred that the composition is a pharmaceutical composition, a food composition, or a food additive composition, wherein, when the composition is a pharmaceutical composition, the pharmaceutical composition is in a dosage form for oral administration or sublingual administration, and, alternatively, when the composition is a food composition, the food composition is a health food, a dietary supplement, a functional food, a nutritional supplement, or a special nutritional food.

Another objective of the present invention is to provide a method for increasing the bioavailability of an active ingredient, comprising incorporating the active ingredient into an oily medium system, wherein
(1) the oily medium system contains a vegetable oil and/or a component of a vegetable oil, but is free of the following substances: phosphate buffered saline (PBS), polyethylene glycols (PEGs), dimethyl sulfoxide (DMSO), ethanol, polypropylene glycols, polysorbates, ethyl acetate, hydroxyethyl 12-hydroxystearate, and tocopheryl polyethylene glycol succinate (TPGS); and
(2) the active ingredient is selected from the group consisting of a compound of formula (I), a pharmaceutically acceptable salts of the compound of formula (I), and combinations thereof, 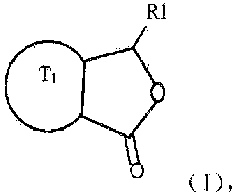 wherein T₁ is a C6 cycloalkane, and R₁ is a C1-C8 aliphatic hydrocarbon group.

Preferably, the combination of the oily medium system and the active ingredient is in aa form as a pharmaceutical composition, a food composition, or a food additive composition. Preferably, when the combination of the oily medium system and the active ingredient is in form as a pharmaceutical composition, the pharmaceutical composition is used for oral administration or sublingual administration; when the combination of the oily medium system and the active ingredient is in form as a food composition, the food composition is a health food, a dietary supplement, a functional food, a nutritional supplement, or a special nutritional food.

In the composition or method according to the present invention, the vegetable oil is preferably selected from the group consisting of sesame oil, perilla oil, sacha inchi oil, brown rice oil, soybean oil, sea buckthorn fruit oil, coconut oil, corn oil, palm oil, peanut oil, grape seed oil, safflower seed oil, pumpkin seed oil, apricot kernel oil, cashew nut oil, hazelnut oil, walnut oil, black bean oil, pine nut oil, black cumin oil, evening primrose oil, sesame leaf essential oil, canola oil, cottonseed oil, olive oil, rapeseed oil, sunflower oil, pecan oil, pistachio oil, castor oil, and combinations thereof. More preferably, the vegetable oil is selected from the group consisting of sesame oil, soybean oil, canola oil, and combinations thereof.

In the composition or method according to the present invention, the active ingredient is preferably selected from the group consisting of a compound of formula (II), a pharmaceutically acceptable salt of the compound of formula (II), a compound of formula (III), a pharmaceutically acceptable salt of the compound of formula (III), and combinations thereof, 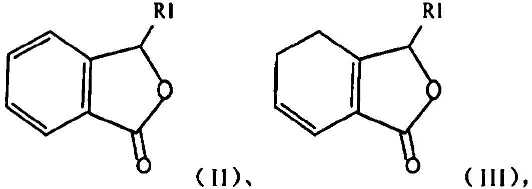 wherein R₁ is a C1-C6 aliphatic hydrocarbon group and preferably is a C4 alkyl or alkenyl group.

In the composition or method according to the present invention, based on the volume of the oily medium system, the concentration of the active ingredient is preferably 0.001 mg/mL to 1,500 mg/mL; furthermore, the volume ratio of the oily medium system to the active ingredient is preferably 1:15 to 30:1.

### BRIEF DESCRIPTION OF THE DRAWINGS

**FIG. 1** is a photograph showing the appearance of compositions according to different embodiments of the present invention after preparation is completed.
**FIG. 2** is a photograph showing the appearance of a composition according to an embodiment of the present invention after being prepared into soft capsules; **FIG. 3A** is an HPLC analysis chromatogram of the contents of the soft capsule when prepared; **FIG. 3B** is an HPLC analysis chromatogram of the contents of the soft capsule after being stored for one year; **FIG. 3C** is an HPLC analysis chromatogram of ACN (i.e., the blank group).
**FIG. 4** is a photograph showing the appearance of compositions according to different embodiments of the present invention after being stored for two years.
**FIG. 5** is a line graph showing the concentration of the active ingredient in the serums sampled from rats at different time points after the composition according to an embodiment of the present invention was administered to the rats.
**FIG. 6** is a line graph showing the concentration of the active ingredient in serums sampled from a human subject at different time points after the composition according to an embodiment of the present invention was administered to the human subjects.
**FIG. 7** is a bar graph showing the relative survival rates of differently treated brain cancer cell lines 1XM (or pancreatic cancer cell lines Mia-PaCa2) compared to the blank group brain cancer cell lines 1XM (or blank group pancreatic cancer cell lines Mia-PaCa2), wherein "***" represents a *p*-value < 0.001 between the two groups.
**FIG. 8** is a bar graph showing the relative survival rates of differently treated brain cancer cell lines 1XM compared to the blank group brain cancer cell lines 1XM, wherein "***" represents a *p*-value < 0.001 between the two groups, "**" represents a *p*-value < 0.01 between the two groups, and "*" represents a *p*-value < 0.05 between the two groups.

### DESCRIPTION OF THE INVENTION

The following will describe the detailed technical content and some specific embodiments of the present invention to enable those skilled in the art to understand the features of the present invention; however, without departing from the spirit of the present invention, the present invention may be practiced in various forms of embodiments, and the scope of protection of the present invention should not be interpreted as being limited to the specific descriptions in the specification.

Unless otherwise indicated herein (especially in the claims), terms such as "a", "an", "the", and similar expressions should be understood to include both the singular and plural forms; the numerical ranges used (such as 5 to 100) should be understood to include all rational numbers within that range and any range composed of any two rational numbers within that range, and thus include all possible combinations of values between the listed minimum and maximum values; "mg/kg body weight" refers to the dosage used per kilogram of individual body weight; the term "individual" refers to a mammalian, including a human or a nonhuman animal; the term bioavailability (BA) refers to an index of the rate and extent to which the active ingredient of a drug (also known as the active component) is absorbed from a formulation into the systemic blood circulation or the site of action.

The bioavailability (BA) of a known active ingredient can be obtained from information provided by a curve graph of the concentration of the active ingredient in the serum of an organism over time after the active ingredient is administered to the organism. In short, the active ingredient is first administered to an organism, and after the administration, serum samples are continuously collected from the organism at specific time intervals; then, the concentration of the active ingredient in the samples is analyzed and a concentration-versus-time curve is plotted, and the Area Under Curve (AUC) is calculated. The value of the AUC is positively correlated with bioavailability-the higher value of AUC, the higher bioavailability of the active ingredient; conversely, a lower value of AUC indicates a lower bioavailability of the active ingredient. Reference of this may be made, for example, to Drug Bioavailability (National Library of Medicine; Gary Price; Deven A. Patel; July 30, 2023), which is entirely incorporated hereinto by reference.

It is known that the compound of formula (I) of the present invention has certain therapeutic benefits for neurological diseases, cerebrovascular diseases, and/or cancers; however, there are still limitations in its application. The primary reason lies in its low bioavailability (AUC < 300 ng* hr/mL), which often necessitates the administration of higher doses to achieve the desired therapeutic effect, leading to an increased risk of systemic side effects and exacerbates the burden on patients. The issues arising from the necessity of increasing dosage due to low bioavailability are particularly severe in the case of oral administration and this can be referred to such asCurr Drug Metab. 2012 Jun 1; 13(5):524-34 and Drug Metab Dispos. 2008 Feb; 36(2):400-8, which is entirely incorporated hereinto by reference.

To address the aforementioned limitations in use, the inventors have discovered through research that the compound of formula (I) and/or a pharmaceutically acceptable salt of the compound of formula (I) has good solubility in the oily medium system of the present invention and can exist stably in the oily medium system for a long period of time (for example, storage at room temperature for up to two years). The inventors have found from further research has found that when the active ingredient of the present invention is administered after being incorporated into the oily medium system, the bioavailability of the active ingredient can be increased, thereby avoiding the issues of requiring increased dosage to provide the desired therapeutic benefit, and alleviating or eliminating unnecessary side effects.

Therefore, the present invention relates to a composition comprising:
(1) an oily medium system, which contains a vegetable oil and/or a component of a vegetable oil, but is free of the following substances: phosphate buffered saline (PBS), polyethylene glycols (PEGs), dimethyl sulfoxide (DMSO), ethanol, polypropylene glycols, polysorbates, ethyl acetate, hydroxyethyl 12-hydroxystearate, and tocopheryl polyethylene glycol succinate (TPGS); and
(2) an active ingredient, which is selected from the group consisting of a compound of formula (I), a pharmaceutically acceptable salt of the compound of formula (I), and combinations thereof, 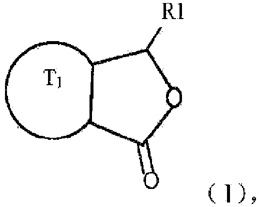 wherein, T₁ is a C6 cycloalkane, and R₁ is a C1-C8 aliphatic hydrocarbon group.

According to some embodiments of the present invention, the composition is used to increase the bioavailability of the active ingredient.

The present invention also relates to a method for increasing the bioavailability of an active ingredient, comprising incorporating the active ingredient into an oily medium system, wherein:
(1) the oily medium system contains a vegetable oil and/or a component of a vegetable oil, but is free of the following substances: phosphate buffered saline (PBS), polyethylene glycols (PEGs), dimethyl sulfoxide (DMSO), ethanol, polypropylene glycols, polysorbates, ethyl acetate, hydroxyethyl 12-hydroxystearate, and tocopheryl polyethylene glycol succinate (TPGS); and
(2) the active ingredient is selected from the group consisting of a compound of formula (I), a pharmaceutically acceptable salt of the compound of formula (I), and combinations thereof, 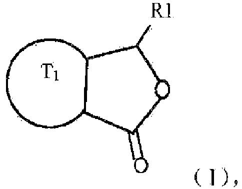 wherein, T₁ is a C6 cycloalkane, and R₁ is a C1-C8 aliphatic hydrocarbon group.

In the composition or method according to the present invention, the vegetable oil involved is preferably selected from the group consisting of sesame oil, perilla oil, sacha inchi oil, brown rice oil, soybean oil, sea buckthorn fruit oil, coconut oil, corn oil, palm oil, peanut oil, grape seed oil, safflower seed oil, pumpkin seed oil, apricot kernel oil, cashew nut oil, hazelnut oil, walnut oil, black bean oil, pine nut oil, black cumin oil, evening primrose oil, sesame leaf essential oil, canola oil, cottonseed oil, olive oil, rapeseed oil, sunflower oil, pecan oil, pistachio oil, castor oil, and combinations thereof; more preferably, the vegetable oil is selected from the group consisting of sesame oil, soybean oil, canola oil, and combinations thereof.

In some embodiments of the composition or method according to the present invention, the oily medium system is composed of the vegetable oil.

In the composition or method according to the present invention, the active ingredient involved is preferably selected from the group consisting of a compounds of formula (II), a pharmaceutically acceptable salt of the compound of formula (II), a compound of formula (III), a pharmaceutically acceptable salt of the compound of formula (III), and combinations thereof, 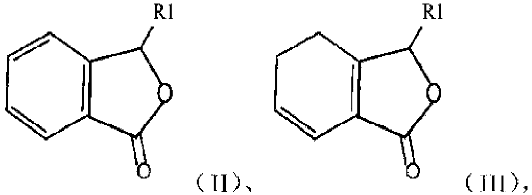 wherein, R₁ is a C1-C6 aliphatic hydrocarbon group and preferably is C4 alkyl or alkenyl group.

In the composition or method according to the present invention, the compound of formula (I) and/or a pharmaceutically acceptable salt of the compound of formula (I) can be purchased from the market, synthesized by a method known in the art of the present invention, or isolated from natural extracts, but is not limited thereto.

In the composition or method according to the present invention, the concentration of the active ingredient in the oily medium system is, in principle, such that no phase separation occurs after the two are mixed. Preferably, based on the weight of the oily medium system, the concentration of the active ingredient can be 50 mg to 500 mg per gram of the oily medium system; based on the volume of the oily medium system, the concentration of the active ingredient can be 0.001 mg to 1,500 mg per milliliter of the oily medium system. In some embodiments of the present invention, the volume ratio of the oily medium system to the active ingredient can be 1:15 to 30:1, such as 1:13, 1:1.5, 1:10, 1:8, 1:6, 1:4, 1:2, 1:1, 2:1, 3:1, 4:1, 5:1, 8:1, 10:1, 13:1, 15:1, 17:1, 20:1, or 25:1. In some embodiments of the present invention, when the oily medium system involved contains sesame oil, based on the volume of the oily medium system, the compound of formula (I) can still exist stably in the oily medium system even as its concentration reaches 920 mg/mL.

The composition of the present invention can be prepared by a simple method of mixing the oily medium system and the active ingredient. In cases where the active ingredient is soluble in the oily medium system, the active ingredient can be directly added to and dissolved in the oily medium system. Conversely, in cases where the active ingredient is insoluble in the oily medium system, physical force (such as shaking or magnetic stirring) can be applied after the active ingredient is added to the oily medium system to provide a stable suspension in which the active ingredient exists in the form of micro-droplets or nano-droplets.

The composition provided according to the present invention can be a pharmaceutical composition, a food composition, or a food additive composition. Furthermore, in the method according to the present invention, the active ingredient and the oily medium system can be combined as a pharmaceutical composition, a food composition, or a food additive composition. In some embodiments of the present invention, the combination of the active ingredient and the oily medium system is provided as a pharmaceutical composition for oral administration.

The pharmaceutical composition provided according to the present invention can be administered systemically or topically, and can be delivered through various drug delivery systems (DDS). For example, the pharmaceutical composition can be delivered via an oral drug delivery system, a transmucosal drug delivery system, a transdermal drug delivery system, and/or an injectable drug delivery system, but is not limited thereto. Furthermore, the pharmaceutical composition can be administered to a subject in need through one or more of the following administration routes: oral administration, sublingual administration, subcutaneous injection, intramuscular injection, intraperitoneal injection, and intravenous injection (including drip infusion and bolus injection), nasal administration, transdermal administration, subcutaneous implantation, and interstitial implantation.

Depending on the form of use and the intended purpose, any pharmaceutically acceptable excipient may be optionally used to provide the pharmaceutical composition, provided that the excipient does not adversely affect the desired benefit(s) of the compound of formula (I) of the present invention and/or the pharmaceutically acceptable salt of the compound of formula (I) and its stability in the oily medium system. Pharmaceutically acceptable excipients known in the art that can be employed include diluents, surfactants, glidants, disintegrants, binders, buffers, colorants, flavoring agents, antioxidants, preservatives, and film-forming agents, etc, but are not limited thereto.

The food composition provided according to the present invention can be a health food, a dietary supplement, a functional food, a nutritional supplement, or a special nutritional food. For example, the food composition can be provided as an oil-based product such as being provided as a capsule containing oil droplets, but is not limited thereto.

Depending on the form and purpose(s) of use the food additive composition provided according to the present invention can be in any suitable form without special limitation, as long as it is in a form convenient for addition during the food manufacturing process. For example, the food additive composition can be provided as an oily food additive such as being provided as an edible essential oil, but is not limited thereto.

The present invention will be further illustrated in detail by the following examples. These examples are provided for illustrative purposes only and are not intended to limit the scope of protection of the present invention. The scope of protection of the present invention is as set forth in the claims.

### Examples

In the following examples, the materials and equipment used are as follows:
1. Black sesame oil: Purchased from I-Mei Foods Co., Ltd., product name: I-Mei 100% Pure Black Sesame Oil.
2. White sesame oil: Purchased from Seitenbacher, product name: Premium Sesame oil.
3. Canola oil: Purchased from TTET UNION CORPORATION.
4. Soybean oil: Purchased from TTET UNION CORPORATION.
5. 2,6-Di-tert-butyl-p-cresol (also known as 2,6-di-tert-butyl-4-methylphenol or Butylated hydroxytoluene, abbreviated as BHT): Purchased from Alfa Aesar.
6.n-butylidenephthalide (BP): Purchased from Penta Manufacturing Company; purity ≥ 97%.
7. (Z)-n-butylidenephthalide (Z-BP): Provided by Everfront Biotech Inc.; Batch No.: F212TR12001; purity 99.8%.
8. Ligustilide (LG): Purchased from Sigma-Aldrich; purity ≥ 96%.
9. Butylphthalide: Purchased from Toronto Research Chemicals; purity ≥ 98%.
10. 1XM cells (a type of human glioblastoma multiforme cell line): Obtained from the research team of Professor Horng-Jyh Harn at Hualien Tzu Chi Hospital (website: https://hlm.tzuchi.com.tw/).
11. Mia-PaCa2 cells (a type of human pancreatic cancer cell line): Obtained from the Bioresource Collection and Research Center (BCRC; website: www.bcrc.firdi.org.tw/); Accession No.: BCRC 60139.
12. 1XM cell culture medium: DMEM culture medium (purchased from Hyclone) supplemented with 10% fetal bovine serum (FBS; purchased from Hyclone) and 1% penicillin/streptomycin (P/S; purchased from Hyclone).
13. Mia-PaCa2 cell culture medium: DMEM/High Glucose culture medium (purchased from Hyclone) supplemented with 10% fetal bovine serum (FBS; purchased from Hyclone), 2.5% horse serum (purchased from Gibco), and 1% penicillin/streptomycin (P/S; purchased from Hyclone).
14. MTT assay kit: Purchased from Sigma-Aldrich.
15. ELISA reader (Enzyme-linked immunosorbent assay analyzer): Purchased from PerkinElmer (USA).

### Example 1 Preparation of the composition of the present invention

### 1-1. Preparation of pharmaceutical composition

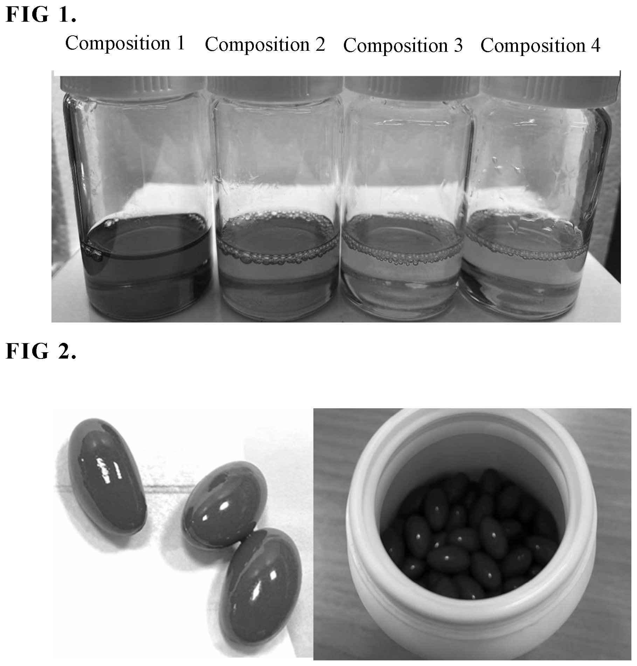

In a glass bottle, components were mixed with the dosage ratios shown in Table 1 to provide a mixture, and the mixture was rapidly stirred with a magnetic stirrer for 20 minutes to form a homogeneous liquid, thereby respectively preparing Compositions 1 to 4. After removing the magnetic stirrer, the glass bottle was tightly sealed and its appearance was observed and photographed, the results are shown in FIG. 1.

**Table 1: Volume Percentages of Composition in Compositions 1 to 4.**

| Composition | 1 | 2 | 3 | 4 |
|---|---|---|---|---|
| BP | 50 | 83.3 | 50 | 83.3 |
| Black sesame oil | 50 | 16.7 | | |
| White sesame oil | | | 50 | 16.7 |

As shown in FIG. 1, no phase separation occurred in any of Compositions 1 to 4, indicating that BP has good solubility in sesame oil. Additionally, Composition 3 was further prepared into soft capsules, and a photograph of the soft capsules is shown in FIG. 2.

### 1-2. Component Analysis

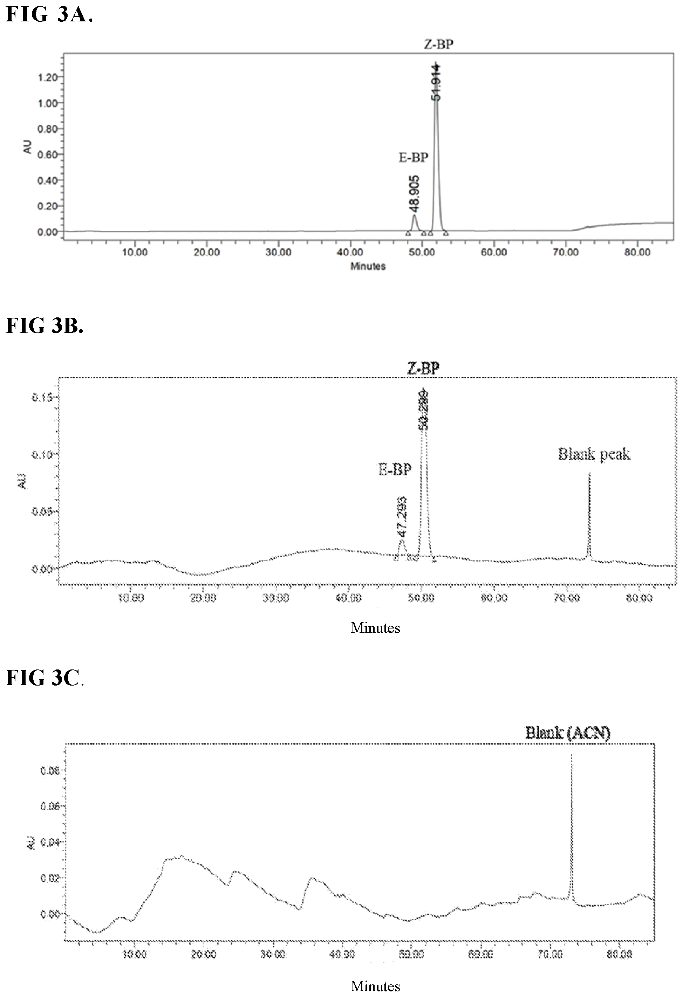

Component analysis was performed on the contents of the soft capsules provided in Example 1-1 at different time points, including respectively at the time when the soft capsules were prepared and after being stored for one year in an environment at room temperature and protected from sunlight. The contents were prepared with acetonitrile (ACN) into samples at a concentration of 1 mg/mL, and the components in the samples were analyzed by High-Performance Liquid Chromatography (HPLC) under the conditions shown in Table 2. The results are shown in FIG. 3A and FIG. 3B, and FIG. 3C is the HPLC analysis chromatogram of ACN. As noted from FIG. 3A to FIG. 3C, even after the soft capsules were stored at room temperature for up to one year, the contents thereof still contained Z-BP and E-BP, indicating that the active ingredient can exist stably in the oily medium system of the present invention for a long period of time.

**Table 2: HPLC Analysis Conditions**

| Analytical instrument | Waters series LC system |
|---|---|
| Column | Sharpsil-U C18 (s-5µm, 100Angstrom, 4.6 x 250mm) with a guard column C18 (5µm, 4.6 x 20mm) |
| Mobile phase condition | 0.1% H₃PO₄ ACN/H₂O (30/70): 0.1% H₃PO₄ ACN = 100: 0 gradient to 5: 95 |
| Temperature | 45 °C |
| Flow rate | 1 mL/min |
| UV detection wavelength | 261 nm |

### Example 2: Stability of the Compositions of the Present Invention

### 2-1. Preparation of Compositions

In a glass bottle, each component was mixed according to the dosage ratios shown in Table 3, and rapidly stirred with a magnetic stirrer for 20 minutes to form a homogeneous liquid, thereby respectively preparing Compositions 5 to 11. After removing the magnetic stirrer, the glass bottle was tightly sealed.

**Table 3: Composition Weight Percentages of Compositions 5 to 11**

| Composition | 5 | 6 | 7 | 8 | 9 | 10 | 11 | 12 |
|---|---|---|---|---|---|---|---|---|
| Figure label | F10319 | F10700 | F10600 | F10900 | F10300 | F1900 | F1300 | D2018-2 |
| BP | 8 | 12 | 15 | 30 | 8 | 30 | 8 | 8 |
| Black sesame oil | 80 | 87 | 85 | 70 | 92 | | | |
| Canola oil | 11.9 | | | | | 70 | 92 | |
| Soybean oil | | | | | | | | 92 |
| BHT | 0.1 | | | | | | | |

### 2-2. Stability Testing

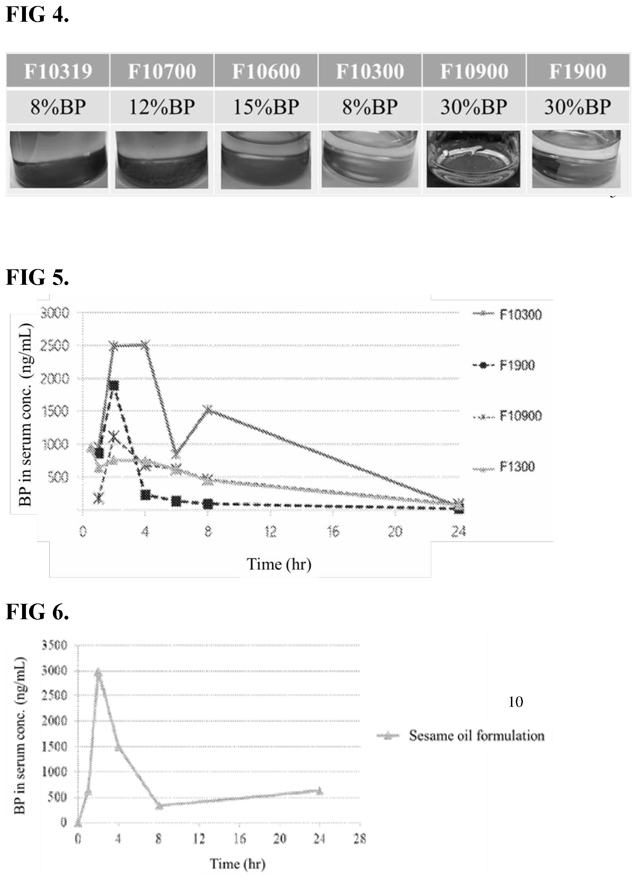

Compositions 5 to 10 provided in Example 2-1 were placed in an environment at room temperature and protected from sunlight for two years, after which their appearance was observed and photographed; the results are shown in FIG. 4.

As noted from FIG. 4, Compositions 5 to 10 did not undergo phase separation even after being stored at room temperature for two years. The aforementioned results demonstrate once again that even after long-term storage, the active ingredient contained in the compositions of the present invention can still exist stably in the oily medium system, exhibiting considerably excellent stability.

### Example 3: Bioavailability Study (Experimental Animals)

### 3-1. Preparation of Serum Samples

After fasting rats for 12 hours, 0.3 mL of blood was drawn from the tail vein of each rat and injected into a blood collection tube containing Potassium (K2) EDTA; subsequently, the blood collection tube was centrifuged for 10 minutes (2000 xg, 2~8°C), and the supernatant (i.e., blank group serum) was collected and frozen at - 70°C for subsequent analysis.

Thereafter, the rats were randomly divided into five groups (2 rats per group) and treated respectively under the following conditions:
1. Group F10900: Administered Composition 8 provided in Example 2-1 via oral feeding at a dosage of 300 mg(BP)/kg body weight.
2. Group F10300: Conducted in the same was as Group F10900, but replacing Composition 8 with Composition 9.
3. Group F1900: Conducted in the same way as Group F10900, but replacing Composition 8 with Composition 10.
4. Group F1300: Conducted in the same way as Group F10900, but replacing Composition 8 with Composition 11.
5. Group D2018-2: Conducted in the same way as Group F10900, but replacing Composition 8 with Composition 12.

At 1, 2, 4, 6, 8, and 24 hours after oral feeding, 0.3 mL of blood was drawn from the tail vein of the rats in each group and injected into blood collection tubes containing EDTA K2 and placed on ice. Subsequently, the blood collection tubes were centrifuged for 10 minutes (2000 xg, 2~8°C), and the supernatants obtained were respectively Group F10300 serum, Group F1900 serum, Group F1300 serum, and Group D2018-2 serum. The serum of each group was frozen at -70°C for subsequent analysis.

### 3-2. Preparation of Analytical Standards

50 µL of a standard solution (containing BP in a concentration ranging from of 0 to 0.5 mg/mL) and 150 µL of the blank group serum mixture provided in Example 3-1 were mixed and then, subjected to ultrasonic vibration for 3 minutes; thereafter, 400 µL of acetonitrile (ACN) was added. After mixing for another 3 minutes with ultrasonic vibration, the mixture was placed in an environment below 0°C for 10 minutes, then returned to room temperature for 5 minutes, and filtered through a 0.2 µm PTFE filter membrane. The resulting filtrate was the analytical standard.

### 3-3. Preparation of Analytical Samples

150 µL of the serum from Group F10300, F1900, F1300, or D2018-2 provided in Example 3-1 and 450 µL of ACN were mixed and then subjected to, of ultrasonic vibration for 3 minutes; thereafter, the mixture was placed in an environment below 0°C for 10 minutes, returned to room temperature for 5 minutes, and filtered through a 0.2 µm PTFE filter membrane. The resulting filtrate was the analytical sample.

### 3-4. HPLC and Pharmacokinetic Analysis

HPLC analysis was performed on the analytical standards and analytical samples provided in Examples 3-2 and 3-3 under the conditions shown in Table 4, and the concentration of BP in the serum samples at each sampling time point was quantified. The results are shown in FIG. 5.

**Table 4: HPLC Analysis Conditions**

| Analytical instrument | Waters series LC system |
|---|---|
| Column | Sharpsil-U C18 (s-5µm, 100Å, 4.6 x 250mm) with a guard column C18 (5µm,4.6 x 20mm) |
| Mobile phase condition | 0.1% H₃PO₄ ACN/H₂O (30/70):0.1% H₃PO₄ ACN = 100:0 gradient to 9:91 |
| Temperature | 40°C |
| Flow rate | 1mL/min |
| UV detection wavelength | 261 nm |

After inputting the obtained BP concentration values at each sampling time point into the Pksolver software, parameters such as Tmax, Cmax, AUC, and T_{1/2}, as well as the bioavailability (BA), were calculated using the NCA IV Bolus model and the linear log Trapezoidal method. The results are shown in Table 5.

**Table 5: Pharmacokinetic Parameters of Each Group**

| Group | Tmax (hours) | Cmax (ng/mL) | AUC (ng×time/mL) | T_{1/2} | BA(%) |
|---|---|---|---|---|---|
| F10900 | 3 | 1506 | 8368 | 6.9 | 13.5 |
| F10300 | 3 | 2902 | 19645 | 4.4 | 31.7 |
| F1900 | 2 | 1891 | 5270 | 7.9 | 9 |
| F1300 | 0.5 | 950 | 8895 | 6 | 14.3 |
| D2018-2 | 1 | 1386 | 2768 | 6.3 | 4.5 |

As noted from FIG. 5 and Table 5, BP was detectable in the serum collected within 24 hours after rats in groups F10300, F1300, F10900, and F1900 were orally administered the compositions of the present invention. Furthermore, the BP content in the serum of groups F10300, F1300, F10900, F1900, and D2018-2 all reached a peak within 3 hours after the rats were orally administered the compositions of the present invention. In addition, the AUC and BA of Group F10300 were higher than those of Group F10900, and the AUC of Group F1300 was higher than that of Group F1900. The aforementioned results demonstrate that the absorption rate and extent of the oral administration of the compositions of the present invention when used in rats are both considerably excellent, and the bioavailability of the active ingredient contained in the compositions of the present invention increases as the proportion of vegetable oil in the composition increases.

The results of this example show that incorporating the compound of formula (I) of the present invention into the oily medium system of the present invention for subsequent administration to a subject individual can improve the bioavailability of the compound of formula (I).

### Example 4: Bioavailability Study (Human Subjects)

### 4-1. Preparation of Serum Samples

1. Blank Group: Before a human subject (weight: 70 kg) orally administered the soft capsules provided in Example 1-1, 5 mL of blood was first drawn and injected into a blood collection tube containing EDTA K2; subsequently, the blood collection tube was centrifuged for 10 minutes (1900 xg, 2~8°C), and the supernatant (i.e., blank group serum) was collected and frozen at -70°C for subsequent analysis.
2. Experimental (Sesame Oil) Group: At 1, 2, 4, 8, and 24 hours after the human subject orally administered the soft capsules provided in Example 1-1 (at a dosage of 4.285 mg(BP)/kg body weight), 5 mL of blood was drawn at each time point and injected into blood collection tubes containing EDTA K2 and placed on ice; subsequently, the blood collection tubes were centrifuged for 10 minutes (1900 xg, 2~8°C), and the supernatants (i.e., serum) were collected and frozen at -70°C for subsequent analysis.

### 4-2. Preparation of Analytical Standards

Conducted in the same way as Example 3-2, but replacing the blank group serum mixture provided in Example 3-1 with the blank group serum provided in Example 4-1 to provide analytical standards.

### 4-3. Preparation of Analytical Samples

Conducted in the same way as Example 3-3, but replacing the serum from groups F10300, F1900, F1300, or F1900 with the "Experimental (Sesame Oil)" group serum provided in Example 4-1 to provide analytical samples.

### 4-4. HPLC Analysis

Analytical standards and analytical samples provided in Examples 4-2 and 4-3 were analyzed in the same way as the method and conditions in Example 3-4, and the concentration of BP in the serum samples at each sampling time point was quantified. The results are shown in FIG. 6.

As noted from FIG. 6, BP was detectable in the serum collected within 24 hours after the human subject orally administered the composition of the present invention, and the BP content in the serum reached a peak about 2 hours after oral administration of the composition of the present invention. The aforementioned results demonstrate that the absorption rate and extent of the composition of the present invention when used for human oral administration are also considerably excellent.

### Example 5: Cancer Cell Cytotoxicity Study I

This example used the MTT assay (3-(4,5-Dimethylthiazol-2-yl)-2,5-diphenyltetrazolium bromide) to investigate the efficacy of serum obtained after administration of the composition of the present invention in killing cancer cells.

Cell cultures in this example were all conducted in an environment of 95% relative humidity, 5% CO2, and 37°C.

### 5-1. Cell Culture

Brain cancer cell lines 1XM (or pancreatic cancer cell lines Mia-PaCa2) were grouped and respectively cultured in 6-well cell culture plates for 48 hours using the following culture media:
1. Blank Group: 1XM cell culture medium (or Mia-PaCa2 cell culture medium).
2. "BP(50)" Group or "BP(100)" Group: 1XM cell culture medium (or Mia-PaCa2 cell culture medium) supplemented with BP (50 or 100 µg/mL).
3. "Blank Serum (Low)", "Blank Serum (Medium)", and "Blank Serum (High)" Groups: 1XM cell culture medium (or Mia-PaCa2 cell culture medium) supplemented with the blank group serum provided in Example 4-1 (at a content in the culture medium of vol. 6.25%, 12.5%, or 25%.
4. "Experimental Serum (Low)", "Experimental Serum (Medium)", and "Experimental Serum (High)" Groups: 1XM cell culture medium (or Mia-PaCa2 cell culture medium) supplemented with the experimental group serum collected at 1, 2, 4, 8, or 24 hours after oral administration of the soft capsules in Example 4-1 (at a content in the culture medium of 6.25, 12.5, or 25 volume percentage).

### 5-2. MTT Assay

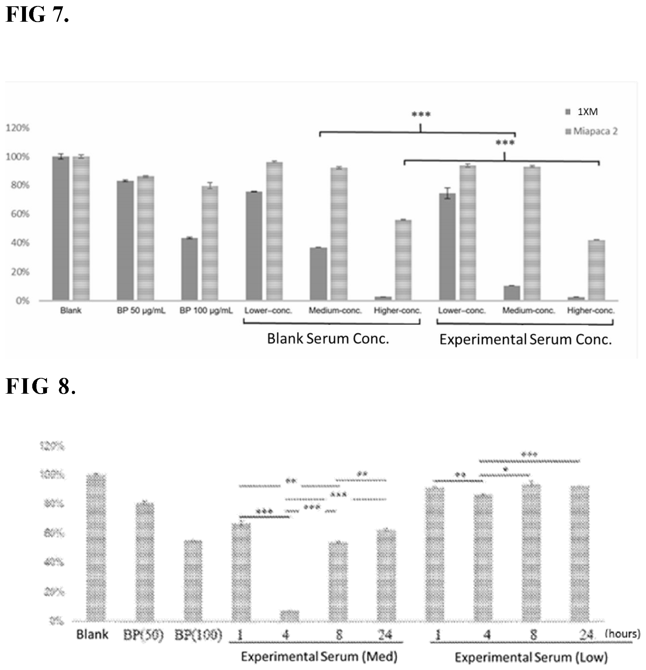

The culture medium in each well of the cell culture plates was removed, and MTT reagent was added (to reach a final concentration of 0.5 mg/mL in each well). The plates were then placed in a 37°C, 5% CO₂ incubator for 2 to 4 hours to form purple needle-like formazan crystals. Subsequently, the solution was aspirated, and 500 µL of dimethyl sulfoxide (DMSO) was added to each well. After shaking with a shaker for 5 to 10 minutes to completely dissolve the purple crystals, the absorbance at a wavelength of 570 or 595 nm was measured using an ELISA reader. The survival rate of the cells was calculated based on the absorbance, with the blank group as the baseline (the survival rate of the blank group was set to 100%). The results are shown in FIG. 7 and FIG. 8. The data of the experimental group serum collected 2 hours after oral administration of the soft capsules are shown in FIG. 7; the data of the experimental group serum collected at 1, 4, 8, or 24 hours are shown in FIG. 8.

As noted from FIG. 7 and FIG. 8, compared to the blank group, the cancer cell survival rates in the "Experimental Serum (Low)", "Experimental Serum (Medium)", and "Experimental Serum (High)" groups were all lower, comparable to the "BP(50)" or "BP(100)" groups. On the other hand, the extent of reduction in cancer cell survival rate followed the order: Experimental Serum (Low) group< Experimental Serum (Medium) group< Experimental Serum (High) group.

The above results demonstrate that the serum obtained after oral administration of the soft capsule containing the compositions of the present invention has the effect of killing brain cancer cells and pancreatic cancer cells. This effect enhances as the concentration of the active ingredient in the serum increases and is comparable to the effect of directly treating cancer cells with the active ingredient. This further confirms that the active ingredient contained in the composition of the present invention possesses excellent bioavailability.

### Example 6: Cancer Cell Cytotoxicity Study II

Cell cultures in this example were all conducted in an environment of 95% relative humidity, 5% CO2, and 37°C.

### 6-1. Cell Culture

Pancreatic cancer cell lines Mia-PaCa2 were grouped and respectively cultured in 96-well cell culture plates for 24 hours using the following culture media:
1. "Z-BP" Group: Mia-PaCa2 cell culture medium supplemented with Z-BP.
2. "Z-BP + Sesame Oil" Group, "Z-BP + Canola Oil" Group, and "Z-BP + Soybean Oil" Group: Mia-PaCa2 cell culture medium supplemented with a mixture prepared by mixing Z-BP with sesame oil, canola oil, or soybean oil in equal proportions.
3. "LG + Sesame Oil" Group, "LG + Canola Oil" Group, and "LG + Soybean Oil" Group: Mia-PaCa2 cell culture medium supplemented with a mixture prepared by mixing LG with sesame oil, canola oil, or soybean oil in equal proportions.

### 6-2. MTT Assay

MTT assay was performed in the same way as the method in Example 5-2, and the concentration value for reaching a 50% fatality rate (inhibitory concentration, IC₅₀) against pancreatic cancer cells was calculated based on the measured survival rates. The results are shown in Table 6.

**Table 6. IC50 Values of Each Group Against Pancreatic Cancer Cells.**

| Group | IC₅₀ (µg/mL) |
|---|---|
| Z-BP | 63.84 |
| Z-BP + sesame oil | 17.35 |
| Z-BP + canola oil | 23.25 |
| Z-BP + soybean oil | 40.66 |
| LG | 44.09 |
| LG +sesame oil | 20.69 |
| LG + canola oil | 25.98 |
| LG + soybean oil | 43.69 |

The results in Table 6 show that incorporating the compound of formula (I) of the present invention into the oily medium system of the present invention increases the efficacy of the compound of formula (I) in killing cancer cells.

As noted from the above Examples, the active ingredient of the present invention (i.e., the compound of formula (I) and/or a pharmaceutically acceptable salt of the compound of formula (I)) has good solubility in the oily medium system of the present invention and can exist stably in the oily medium system for a long period of time. Furthermore, the bioavailability of the active ingredient of the present invention can be increased by incorporating the active ingredient into an oily medium system before administration, thereby avoiding the problem of requiring increased dosage to provide the desired therapeutic benefit(s), and alleviating or eliminating unnecessary side effect(s).

## Claims

1. A composition, **characterized in** comprising:
(1) an oily medium system, which contains a vegetable oil and/or a component of a vegetable oil, but is free of the following substances: phosphate buffered saline(PBS), polyethylene glycols (PEGs), dimethyl sulfoxide (DMSO), ethanol, polypropylene glycols, polysorbates, ethyl acetate, hydroxyethyl 12-hydroxystearate, and tocopheryl polyethylene glycol succinate (TPGS); and
(2) an active ingredient, which is selected from the group consisting of: a compound of formula (I), a pharmaceutically acceptable salt of the compound of formula (I), and combinations thereof, 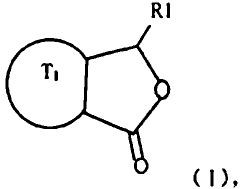 wherein,
T1 is a C6 cycloalkane; and
R1 is a C1-C8 aliphatic hydrocarbon group.

2. The composition according to claim 1, **characterized in that** the vegetable oil is selected from the group consisting of sesame oil, perilla oil, sacha inchi oil, brown rice oil, soybean oil, sea buckthorn fruit oil, coconut oil, corn oil, palm oil, peanut oil, grape seed oil, safflower seed oil, pumpkin seed oil, apricot kernel oil, cashew nut oil, hazelnut oil, walnut oil, black bean oil, pine nut oil, black cumin oil, evening primrose oil, sesame leaf essential oil, canola oil, cottonseed oil, olive oil, rapeseed oil, sunflower oil, pecan oil, pistachio oil, castor oil, and combinations thereof .

3. The composition according to claim 1, **characterized in that** the vegetable oil is selected from the group consisting of sesame oil, soybean oil, canola oil, and combinations thereof.

4. The composition according to claim 1, **characterized in that** the active ingredient is selected from the group consisting of: a compound of formula (II), a pharmaceutically acceptable salt of the compound of formula (II), a compound of formula (III), a pharmaceutically acceptable salt of the compound of formula (III), and combinations thereof, 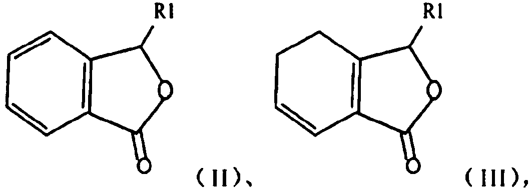 wherein R1 is a C1-C6 aliphatic hydrocarbon group.

5. The composition according to claim 4, **characterized in that** R1 is a C4 alkyl group or an alkenyl group.

6. The composition according to any one of claims 1 to 5, **characterized in that**, based on the volume of the oily medium system, the concentration of the active ingredient is 0.001 mg/mL to 1,500 mg/mL.

7. The composition according to any one of claims 1 to 5, **characterized in that** the volume ratio of the oily medium system to the active ingredient is 1:15 to 30:1.

8. The composition according to any one of claims 1 to 5, **characterized in that** the composition is used to increase the bioavailability of the active ingredient.

9. The composition according to claim 8, **characterized in that** the composition is a pharmaceutical composition, a food composition, or a food additive composition.

10. The composition according to claim 9, **characterized in that** the composition is a pharmaceutical composition in an oral or sublingual drug delivery form.

11. The composition according to claim 9, **characterized in that** the composition is a food composition, which is a health food, a dietary supplement, a functional food, a nutritional supplement, or a special nutritional food.

12. A method for increasing the bioavailability of an active ingredient, **characterized in** comprising incorporating the active ingredient into an oily medium system, wherein:
(1) the oily medium system contains a vegetable oil and/or a component of a vegetable oil, but is free of the following substances: phosphate buffered saline (PBS), polyethylene glycols (PEGs), dimethyl sulfoxide (DMSO), ethanol, polypropylene glycols, polysorbates, ethyl acetate, hydroxyethyl 12-hydroxystearate, and tocopheryl polyethylene glycol succinate (TPGS); and
(2) the active ingredient is selected from the group consisting of a compound of formula (I), a pharmaceutically acceptable salt of the compound of formula (I), and combinations thereof, 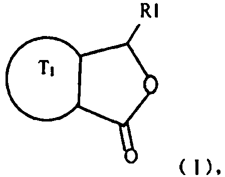 wherein,
T1 is a C6 cycloalkane; and
R1 is a C1-C8 aliphatic hydrocarbon group.

13. The method according to claim 12, **characterized in that** the vegetable oil is selected from the group consisting of sesame oil, perilla oil, sacha inchi oil, brown rice oil, soybean oil, sea buckthorn fruit oil, coconut oil, corn oil, palm oil, peanut oil, grape seed oil, safflower seed oil, pumpkin seed oil, apricot kernel oil, cashew nut oil, hazelnut oil, walnut oil, black bean oil, pine nut oil, black cumin oil, evening primrose oil, sesame leaf essential oil, canola oil, cottonseed oil, olive oil, rapeseed oil, sunflower oil, pecan oil, pistachio oil, castor oil, and combinations thereof.

14. The method according to claim 12, **characterized in that** the vegetable oil is selected from the group consisting of sesame oil, soybean oil, canola oil, and combinations thereof.

15. The method according to claim 12, **characterized in that** the active ingredient is selected from the group consisting of a compound of formula (II), a pharmaceutically acceptable salt of the compound of formula (II), a compound of formula (III), a pharmaceutically acceptable salt of the compound of formula (III), and combinations thereof, 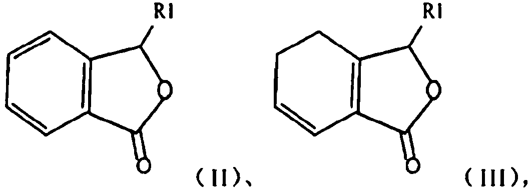 wherein, R1 is a C1-C6 aliphatic hydrocarbon group.

16. The method according to claim 15, **characterized in that** R1 is a C4 alkyl group or an alkenyl group.

17. The method according to any one of claims 12 to 16, **characterized in that**, based on the volume of the oily medium system, the concentration of the active ingredient is 0.001 mg/mL to 1,500 mg/mL.

18. The method according to any one of claims 12 to 16, **characterized in that** the volume ratio of the oily medium system to the active ingredient is 1:15 to 30:1.

19. The method according to any one of claims 12 to 16, **characterized in that** the oily medium system and the active ingredient are combined in a form of a pharmaceutical composition, a food composition, or a food additive composition.

20. The method according to claim 19, **characterized in that** when the combined oily medium system and active ingredient are in the form of a pharmaceutical composition, the pharmaceutical composition is used for oral or sublingual administration.

21. The method according to claim 19, **characterized in that** when the combined oily medium system and active ingredient are in the form of a food composition, the food composition is a health food, a dietary supplement, a functional food, a nutritional supplement, or a special nutritional food.
